Europäisches Patentamt

European Patent Office

Office européen des brevets

(11) Numéro de publication: **0 003 088**
**B1**

# FASCICULE DE BREVET EUROPEEN

(45) Date de publication du fascicule de brevet: **16.09.81**

(51) Int. Cl.³: **G 01 N 33/00, A 61 B 5/08**

(21) Numéro de dépôt: **78400261.0**

(22) Date de dépôt: **22.12.78**

(54) **Olfactomètre différentiel.**

(30) Priorité: **10.01.78 FR 7800509**

(43) Date de publication de la demande:
**25.07.79 Bulletin 79/15**

(45) Mention de la délivrance du brevet:
**16.09.81 Bulletin 81/37**

(84) Etats Contractants Désignés:
**BE CH DE GB IT NL SE**

(56) Documents cités:
**FR - A - 2 015 152
FR - A - 2 210 298
FR - A - 2 212 938
US - A - 2 327 060
US - A - 2 950 618**

(73) Titulaire: **COMMISSARIAT A L'ENERGIE
ATOMIQUE Etablissement de Caractère
Scientifique Technique et Industriel
B.P. 510
F-75752 Paris Cedex 15 (FR)**

(72) Inventeur: **Chuiton, René
8, Avenue M. Renaudin
F-92140 Clamart (FR)**
Inventeur: **Mac Leod, Patrick
424, Avenue de la Division Leclerc
F-92290 Chatenay-Malabry (FR)**

(74) Mandataire: **Mongredien, André et al,
c/o Brevatome 25, rue de Ponthieu
F-75008 Paris (FR)**

Courier Press, Leamington Spa, England.

## Olfactomètre différentiel

La présente invention concerne un olfactomètre différentiel. Elle se rapporte à un détecteur de zéro physiologique pour mesurer les intensités odorantes de stimuli olfactifs distincts.

On sait que l'olfactométrie a pour but de délivrer un stimulus olfactif bien défini en composition chimique et en concentration. Elle fait appel à des dispositifs capables de diluer un ou plusieurs gaz odorants dans un gaz inodore, tel que l'air ou l'azote.

L'olfactométrie répond à deux préoccupations essentielles: la mesure du seuil olfactif (olfactométrie liminaire), ou la mesure des intensités odorantes (olfactométrie supraliminaire).

L'olfactométrie liminaire est simple. En effet, il est facile de mesurer un seuil; la méthode expérimentale consiste à rechercher la plus faible concentration que le sujet est capable de percevoir avec une probabilité de 50%.

L'olfactométrie supraliminaire est plus délicate. Deux méthodes peuvent être utilisées:

—une méthode d'estimation subjective de l'intensité, exprimée par un chiffre compris entre 0 et un maximum,

—une méthode consistant à établir l'égalisation entre le stimulus expérimental et un stimulus étalon, convenablement choisi dans une gamme d'intensité; cette égalisation se fait par approximations successives.

Un autre problème, difficile à résoudre en olfactomètre, est celui de la dilution et du mode de présentation du stimulus. Cette dilution peut être statique ou dynamique, tandis que la présentation du stimulus peut être active (par inhalation ou par flairage), ou passive (par injection dans les fosses nasales du sujet).

La dilution statique est obtenue en introduisant une quantité connue de substances odorantes dans un volume connu de gaz inodore, contenu dans une enceinte inerte. La dilution dynamique est obtenue en réalisant le mélange d'un faible courant gazeux odorant, avec un fort courant gazeux inodore. En multipliant les étages de dilution, une concentration quelconque peut ainsi être obtenue. les meilleurs résultats sont obtenus en utilisant des olfactomètres dynamiques à présentation active du stimulus. Dans le cas de l'olfactométrie supraliminaire, la méthode d'égalisation est celle qui donne les résultats les plus précis et reproductibles.

Toutefois, même en se plaçant dans les conditions optimales qui viennent d'être définies, les mesures olfactométriques souffrent des inconvénients suivants:

—les appareils sont lourds, encombrants, fragiles et d'un maniement délicat;

—les mesures d'intensité sont limitées en précision par une donnée physiologique: la plus petite différence d'intensité perceptible, correspond à une augmentation de concentration de l'ordre de 50% (ou à une diminution de l'ordre de 33%).

Des résultats reproductibles ne sont obtenus qu'en répétant les mesures plusieurs fois, sur plusieurs sujets différents, de façon à obtenir des valeurs moyennes excluant les variations interindividuelles, aussi bien que les variations intra-individuelles. Il en résulte qu'il est impossible de mesurer avec certitude l'intensité odorante d'un stimulus donné, pour un sujet donné, à un instant donné.

Chez l'homme et tous les animaux à organes olfactifs pairs, les sensations reçues par la muqueuse olfactive d'un côté, sont transmises au bulbe olfactif du même côté. Les deux bulbes olfactifs sont reliés par un circuit nerveux qui établit entre eux une inhibition réciproque. Plus l'un des bulbes est activé, plus il freine la réponse de l'autre. Cette inhibition réciproque est maximale lorsque les deux stimuli sont parfaitement synchrones, à une milliseconde près. Elle crée entre les deux bulbes olfactifs une situation d'équilibre instable, que amplifie les différences, d'autant plus efficacement qu'elles sont plus faibles. L'égalité d'intensité de deux stimuli peut donc être appréciée avec une grande précision; l'ensemble des deux bulbes se comporte alors comme un détecteur de zéro à gain élevé.

On sait réaliser un dispositif permettant de surmonter les inconvénients mentionnés plus haut, et notamment un dispositif de maniement facile, permettant d'obtenir des mesures précises et reproductibles. Ces buts sont atteints par détection du zéro physiologique en mesurant l'égalité des intensités odorantes de deux stimuli olfactifs distincts présentés simultanément chacun d'un côté du nez du même sujet.

On opère de la manière suivante: on présente simultanément, par l'intermédiaire d'une commande électronique déclenchée par l'expérimentateur, deux stimuli olfactifs, l'un étalon et l'autre à mesurer, chacun respectivement à une narine du nez d'un même expérimentateur. On compare ensuite les intensités respectives des stimuli, par la perception la plus forte ressentie dans une narine. Enfin, on égalise les intensités dans chaque narine en réglant l'amplitude du stimulus à mesurer, par rapport au stimulus étalon. Les deux stimuli doivent être d'une part, rigoureusement synchrones, et d'autre part, séparément ajustables en amplitude. De plus, pour éliminer les distorsions éventuellement introduites par une asymétrie des fosses nasales, il est nécessaire de procéder comme pour une double pesée: soit par exemple, un stimulus étalon E et un stimulus X, dont on veut ajuster l'amplitude pour que son intensité égale celle de E. On présente d'abord E à droite et on l'égalise avec un stimulus accessoire R, présenté à gauche et jouant le rôle d'une tare. Sans modifier R, on présente ensuite X à droite et on égalise avec R. On peut alors dire que E et X ont la même intensité odorante.

L'égalité des sensations peut être appréciée electro-physiologiquement chez l'animal ou subjectivement chez l'homme. Dans le premier cas, on utilise les signaux recueillis par des électrodes judicieusement placées dans les deux bulbes olfactifs. Dans le deuxième cas, on a recours à l'expression verbale pour indiquer le côté où la sensation est perçue de la manière la plus forte. On dit que les intensités comparées sont égales lorsque la sensation est perçue, soit "au milieu", soit pas plus fort "d'un côté que de l'autre".

D'apres le brevet français No. 2210298 déposé le 8 Décembre 1972 au nom du même demandeur, en connait un olfactomètre différentiel permettant d'effectuer des mesures olfactométriques supraliminaires, qui comprend:

—deux injecteurs d'odeur avec des gicleurs (2a, 3a) montés sur un support (6, 6a) de centrage nasal;

—un dispositif antidiffusion (12) escamotable destiné à occulter les gicleurs pendant les intervalles entre les périodes de stimulation;

—une unité de commande électronique (15) et un dispositif de détection de la respiration (16) pour commander les gicleurs.

Chaque injecteur comporte un gicleur qui est monté sur un réservoir déformable sous l'action d'une transmission élastique commandée par un moteur.

Un tel olfactomètre différentiel permet d'effectuer des mesures olfactométriques supraliminaires mais il a pour inconvénient de présenter une commande pulsée d'alimentation des gicleurs par un réservoir déformable; cette commande pulsée doit intervenir en synchronisme avec une commande d'escamotage du dispositif antidiffusion. De plus, la commande du dispositif antidiffusion est réalisée par l'intermédiaire du dispositif de détection de la respiration; ce dispositif de détection comporte un seul capteur placé au voisinage de l'un des gicleurs. Il en résulte qu'en cas d'une forte dissymétrie dans le pouvoir d'inspiration des narines de l'expérimentateur, le déclenchement de l'injection des stimuli olfactifs n'est pas effectué à l'instant le plus approprié aux mesures à effectuer.

L'invention a pour but de remédier à ces inconvénients, et notamment de réaliser un olfactomètre différentiel dans lequel le stimulus parvient au sujet par voie dynamique, et dans lequel le déclenchement de l'injection des stimuli olfactifs se fait à l'instant le plus approprié aux mesures olfactométriques.

L'invention a pour objet un olfactomètre différentiel comprenant:

—deux gicleurs situés de part et d'autre d'un support nasal et respectivement associés à un circuit d'alimentation en stimuli olfactifs;

—un dispositif antidiffusion escamotable destiné à occulter les gicleurs pendant des intervalles de temps compris entre les périodes de stimulation nasale;

—un dispositif de détection de la respiration associé à une unité de commande électronique pour commander l'occultation des gicleurs;

—des moyens pour alimenter les gicleurs en permanence.

Caractérisé en ce que le dispositif antidiffusion est constitué, pour chaque gicleur, par une gaine entourant le conduit, cette gaine présente à sa partie supérieure, du côté de l'orifice du conduit, un orifice permettant le passage de ce conduit, tandis que sa partie inférieure évasée par rapport au conduit débouche dans une chambre de pompage permanent; cette gaine est associée à l'unité de commande électronique par des moyens pour la déplacer de manière linéaire, le long du conduit, de manière que le conduit soit entièrement contenu dans la gaine lorsque le détecteur de coïncidence ne délivre aucun signal de commande et que le conduit émerge de l'orifice de la gaine, lorsque le détecteur de coïncidence délivre un signal de commande d'escamotage.

Selon une caractéristique avantageuse, le dispositif de détection de respiration comprend:

—deux capteurs de température disposés respectivement au voisinage de chaque gicleur et associés à l'unité de commande électronique d'occultation des gicleurs;

—un dispositif détecteur de coïncidence connecté aux deux capteurs et à l'unité de commande électronique qui commande l'escamotage du dispositif antidiffusion lorsque les capteurs présentent un refroidissement simultané.

Selon une caractéristique particulière, chacun des gicleurs est constitué par un conduit à orifice calibré.

D'autres caractéristiques et avantages de l'invention ressortiront encore de la description détaillée qui va suivre, donnée à titre purement illustratif, en référence aux dessins annexés, dans lesquels:

—la figure 1 représente un olfactomètre conforme à l'invention, selon un premier mode de réalisation;

—la figure 2 représente un olfactomètre conforme à l'invention, selon un deuxième mode de réalisation;

—la figure 3 représente le dispositif de détection de la respiration, associé à l'unité de commande électronique permettant de commander l'occultation des gicleurs;

—la figure 4 est un diagramme représentant des signaux apparaîssant en certains points de l'unité de commande de la figure 3;

—la figure 5 est une vue en coupe latérale du support nasal à position réglable;

—la figure 6 représente une partie d'un circuit d'alimentation, pouvant être associée à plusieurs olfactomètres.

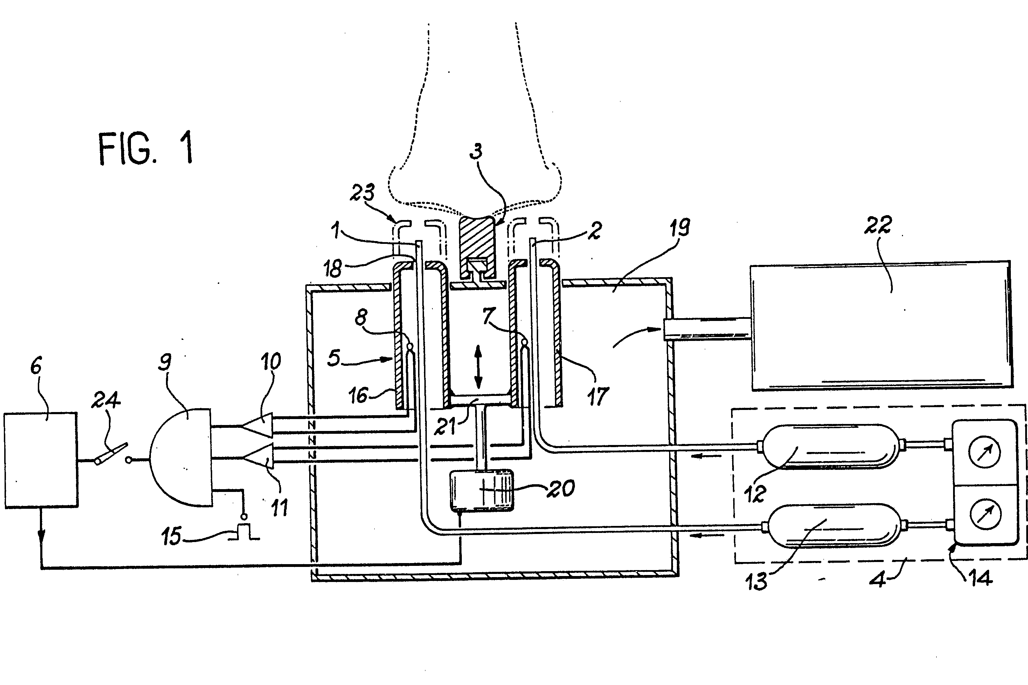

En référence à la figure 1, on a représenté un premier mode de réalisation d'un olfactomètre conforme à l'invention. Cet olfactomètre comprend deux gicleurs 1, 2 situés de part et d'autre d'un support nasal 3. Ces gicleurs sont associés à un circuit d'alimentation 4, en stimuli olfactifs. Un dispositif antidiffusion, escamotable 5, est associé aux gicleurs, de manière à occulter ceux-ci pendant des intervalles de temps compris entre les périodes de stimulation nasale. Une unité de commande électronique 6 associée à une dispositif de détection de la respiration permet de commander l'occultation des gicleurs; cette unité de commande électronique est constituée par des capteurs de température 7, 8 agissant sur un détecteur de coïncidence 9, par l'intermédiaire d'amplificateurs 10, 11. Le circuit 4 d'alimentation des gicleurs comprend des moyens pour alimenter ceux-ci en permanence. Ces moyens sont constitués par des cartouches à odeurs 12, 13, contenant chacune un produit odorant ou un mélange odorant. La cartouche 12, par exemple, peut être celle qui est choisie pour référence. Chacune de ces cartouches débouche respectivement sur un gicleur et sur des moyens 14 d'alimentation par un fluide gazeux à pression réglable. Ce fluide gazeux est par exemple de l'air ou de l'azote sous pression. Les cartouches peuvent être en verre ou en métal et le produit odorant ou le mélange odorant est généralement contenu dans un corps poreux situé à l'intérieur des cartouches. Le débit des stimuli olfactifs est réglé d'une part par la pression du fluide gazeux délivré par les moyens 14, et, d'autre part, grâce au calibrage de l'orifice des gicleurs, 1, 2. Les capteurs de température 7, 8 sont disposés respectivement au voisinage de chaque gicleur et le détecteur de coïncidence 9 délivre, à l'unité de commande électronique 6, un signal de commande d'escamotage du dispositif antidiffusion 5, lorsque ces capteurs présentent un refroidissement simultané. Ce refroidissement simultané apparaît lorsque l'expérimentateur, dont le nez repose sur le support nasal 3 inspire, au-dessus des gicleurs. La simultanéité de ce refroidissement est repérée grâce au détecteur de coïncidence 9; ce détecteur de coïncidence est une porte logique de type ET, à deux entrées; chaque entrée reste active pendant quelques millisecondes et quand les deux entrées sont actives simultanément, un signal est transmis à l'unité de commande électronique 6 qui délivre le signal de commande d'escamotage du dispositif antidiffusion 5.

Le dispositif antidiffusion 5 comprend pour chacun des gicleurs, des gaines 16, 17 qui entourant les conduits formés par les gicleurs. Chaque gaine présente à sa partie supérieure, un orifice 18, permettant le passage du conduit. La partie inférieure de chaque gaine est évasée par rapport au conduit et débouche dans une chambre 19, de pompage permanent. Le dispositif antidiffusion 5 comprend également des moyens qui permettent de déplacer linéairement les gaines le long des conduits des injecteurs 1, 2 de manière que le conduit soit entièrement contenu dans la gaine, lorsque le détecteur de coïncidence ne délivre aucun signal de commande; ce conduit émerge de l'orifice de la gaine lorsque le détecteur de coïncidence délivre un signal de commande d'escamotage. Dans ce premier mode de réalisation de l'olfactomètre de l'invention, les moyens qui permettent de déplacer les gaines comprennent un moteur 20, de type électromagnétique à déplacements linéaires alternatifs. Ce moteur, de type connu, n'est pas représenté en détail sur la figure; il est associé à un élément 21 de couplage des gaines et il est commandé par l'unité de commande électronique 6. La chambre 19 de pompage est bien entendu reliée à une pompe 22 qui permet de pomper en permanence, dans le volume des gaines, de manière à éviter toute propagation du stimulus lorsque les gaines sont en position haute.

Le fonctionnement de cet olfactomètre est alors le suivant: le stimulus de référence étant placé dans la cartouche 12, par exemple, et le gaz odorant à tester dans la cartouche 13, l'expérimentateur place son nez sur le support nasal 3 et ferme l'interrupteur 24 qui permet de relier la sortie du détecteur de coïncidence 9 à l'entrée de l'unité de commande électronique 6. Les gaines 16, 17 sont en position haute 23, au départ. Dès que l'expérimentateur inspire, il y a refroidissement des capteurs 7, 8 et si ce refroidissement se produit quasi simultanément à 5 millisecondes près par exemple, la porte ET 9 est passante et un signal de commande d'escamotage des gaines 16, 17 du dispositif antidiffusion, parvient à l'unité de commande électronique 6. Cette unité de commande électronique 6, comme on le verra par la suite, comprend des moyens qui permettent de fixer un délai réglable pour l'application au moteur 20 d'un signal de commande d'escamotage. Cette unité de commande comprend également des moyens pour fixer une durée d'escamotage des gaines, à partir de la fin de ce délai. A titre d'exemple, le retard introduit dans l'escamotage des gaines est d'environ 100 millisecondes, tandis que la durée d'escamotage est de 50 millisecondes. Comme on le verra plus loin en détail, ce délai et cette durée permettent de réaliser une mesure olfactométrique au moment où la sensibilité olfactive de l'expérimentateur est maximale. Lorsque les gaines sont ainsi escamotées, le stimulus de référence et le gaz odorant à tester parviennent respectivement à chacune des narines de l'expérimentateur. Il suffit alors de régler la pression du gaz inodore injecté dans la cartouche de test 13, jusqu'à l'obtention d'une sensation d'intensité odorante équivalente dans chacune des narines. L'expérimentateur peut alors inverser les cartouches de test et de référence et recommencer la même opération. L'intensité odorante perçue par l'expérimentateur, dans chacune de ses narines, peut alors être exprimée en fonction de la pression du gaz inodore délivré par les moyens 14 qui alimentent en gaz inodore chacune des cartouches. Comme on l'a vu précédemment, l'expérimentateur peut également procéder à une mesure olfactométrique en utilisant la méthode de double pesée. Lorsque les gaines 16, 17 sont en position haute 23, les moyens de pompage 22 évacuent les stimuli olfactifs au-dessus des gicleurs 1, 2 de sorte que l'expérimentateur n'a aucune perception de ces stimuli.

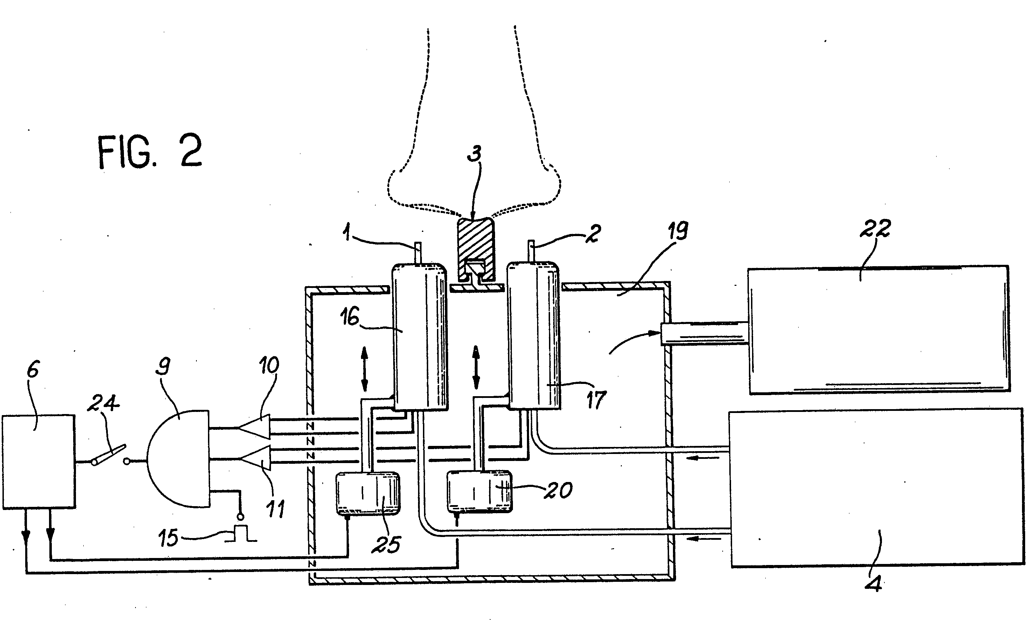

En référence à la figure 2, on a représenté un autre mode de réalisation de l'olfactomètre de l'invention. Les mêmes éléments portent les mêmes références sur les figures 1 et 2. Selon ce deuxième mode de réalisation de l'olfactomètre de l'invention, les moyens qui permettent de déplacer linéairement les gaines le long des gicleurs 1, 2 comprennent pour chacun des gicleurs, un moteur de type électromagnétique à déplacements linéaires alternatifs. Ces moteurs sont représentés en 20 et 25. Comme précedemment, chacun de ces moteurs reçoit un signal de commande de l'unité de commande électronique 6. Le signal de commande reçu par chacun des moteurs est bien entendu, appliqué avec un délai et persiste pendant une durée prédéterminée. Le principe des mesures olfactométriques n'est pas différent du principe utilisé dans le premier mode de réalisation de l'olfactomètre. Les différences entre ces deux modes de réalisation portent essentiellement sur la présence de deux moteurs 20, 25 et qui permettent une commande séparée de l'escamotage des gaines. Il existe également une différence au niveau de l'unité de commande électronique 6 qui sera décrite plus loin en détail.

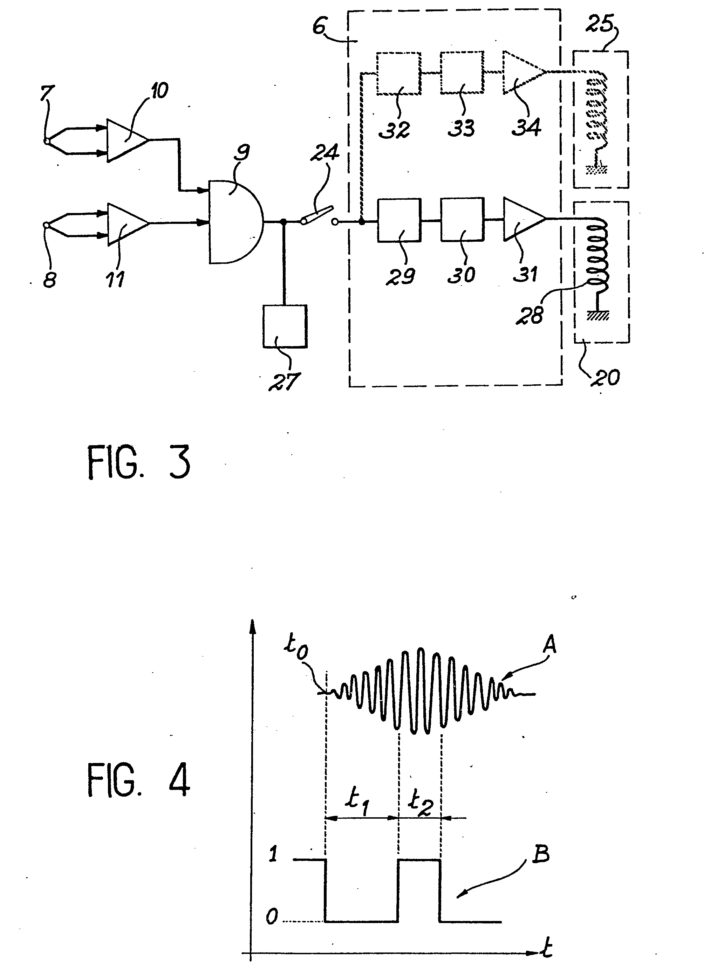

En référence à la figure 3, on a représenté l'unité de commande électronique 6, associée au détecteur de coïncidence 9 constitué par une porte ET; cette porte permet de détecter la simultanéité des refroidissements des capteurs thermiques 7 et 8 dont les signaux de sortie agissent sur les entrées de cette porte par l'intermédiaire des amplificateurs 10 et 11; lorsqu'un refroidissement simultané des capteurs thermiques 7 et 8 est détecté, un signal de commande est envoyé à l'unité de commande électronique 6. Ce refroidissement peut être perçu par l'expérimentateur grâce à un témoin visuel ou sonore 27. Il est bien entendu que la mise en oeuvre d'une mesure olfactométrique ne peut être réalisée que grâce à la fermeture de l'interrupteur 25. Dans le premier mode de réalisation de l'olfactomètre de l'invention décrit en référence à la figure 1, le signal de commande agit de manière à provoquer l'escamotage des gaines 16 et 17, grâce au moteur à déplacements linéaires 20. Ce moteur peut être semblable au moteur que l'on utilise dans les haut-parleurs; on a représenté simplement sur cette figure une bobine 28 qui se déplace dans une culasse aimantée. C'est cette bobine qui commande le déplacement linéaire alternatif des gaines 16, 17. Dans le premier mode de réalisation de l'invention, l'unité de commande électronique comprend des moyens 29 permettant de retarder d'un certain délai le signal de commande, et comprend des moyens 30 permettant de fixer une durée d'application du signal de commande au moteur 20. Les moyens 29 qui permettent de fixer un délai d'application du signal de commande, sont constitués par une bascule monostable dont la période peut être fixée, par exemple, à 100 millisecondes. Les moyens 30 qui permettent de fixer une durée d'application du signal de commande sont également constitués par une bascule monostable ayant par exemple une période de 50 millisecondes. Cette bascule monostable 30 est associée à un amplificateur 31, dont la sortie commande la bobine 28 du moteur 20. Dans le second mode de réalisation de l'olfactomètre de l'invention, l'escamotage des gaines 16, 17 est commandé séparément par les moteurs 20 et 25. Il en résulte que l'unité de comande électronique présente une seconde voie de commande comprenant comme précédemment des moyens pour fixer un délai et une durée d'application du signal de commande, à un autre moteur 25. Ces moyens sont, comme précédemment, constitués par une bascule monostable 32, permettant de fixer le délai d'application du signal de commande et par une bascule monostable 33, permettant de fixer la durée d'application de ce signal au moteur 25, par l'intermédiaire d'un amplificateur 34. En principe, les délais et les durées sur les deux voies de commande 20 et 25 sont respectivement identiques, mais il est bien évident que ces délais et ces durées peuvent être réglables en fonction des mesures et des études que veut effectuer l'expérimentateur.

En référence à la figure 4, on a représenté en fonction du temps t, le diagramme A montrant l'évolution de l'amplitude d'une inspiration. On a représenté en B les signaux obtenus en sortie des bascules monostables 29 et 30 de la figure 3. Au moment d'une inspiration, le signal de commande émis par la porte ET 9, parvient, à l'instant $t_0$ à la bascule monostable 29 permettant de fixer le délai d'application du signal de commande au moteur 20. Ce délai de durée $t_1$ peut, par exemple, être égal à 100 millisecondes. Il permet d'appliquer le signal de commande d'escamotage des gaines à l'instant où l'inspiration représentée sur le diagramme A présente une amplitude maximale. La fin de la période de cette bascule monostable 29 correspond à l'actionnement de la bascule monostable 30, qui permet de fixer la durée $t_2$ d'application du signal de commande au moteur 20. Cette durée peut, par exemple, être de 50 millisecondes. Elle permet un escamotage des gaines durant un intervalle de temps qui correspond à une amplitude maximale de l'inspiration.

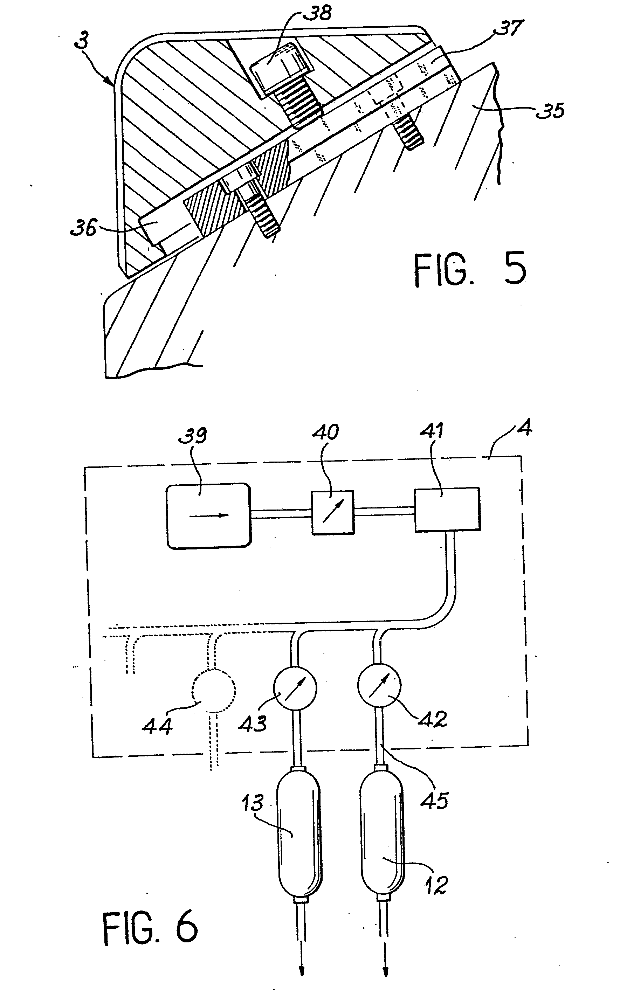

En référence à la figure 5, on a représenté le support nasal 3 de la figure 1. Ce support est représenté en coupe latérale. Il est disposé sur un socle incliné 35 de l'olfactomètre. Une rainure 36 en forme de T peut coulisser le long d'une réglette 37, également en forme de T. Le support 3 est maintenu sur cette réglette grâce à une vis 38 par exemple. Ce déplacement du support 3 sur ce plan incliné du socle 35 permet de positionner la hauteur du nez de l'expérimentateur par rapport aux orifices des gicleurs 1 et 2.

En référence à la figure 6, on a représenté une partie du circuit d'alimentation 4, pouvant être associé à plusieurs olfactomètres. Ce circuit d'alimentation comprend une source 39 d'air comprimé ou de gaz inodore sous pression, associée à un régulateur de pression 40. La sortie de ce régulateur alimente un filtre 41 désordorisant, au charbon actif. Il est possible de connecter, à la sortie de ce filtre, des cartouches à odeur 12, 13, etc... alimentant plusieurs olfactomètres. La pression de l'air comprimé ou du gaz inodore dans ces cartouches peut être réglée grâce à des manomètres 42, 43, 44, etc... Les tubulures telles que 45 reliant le manomètre aux cartouches peuvent également permettre de régler le débit du fluide gazeux injecté dans ces cartouches; en effet, il suffit pour cela de choisir en fonction du débit désiré la longueur ou la section de ces tubulures.

L'olfactomètre qui vient d'être décrit permet des mesures d'intensité odorantes, sûres et rapides, à partir d'une évaluation synchrone. Le stimulus de référence et le gaz odorant à tester sont libérés simultanément dans les deux narines. Cet olfactomètre permet de tirer le plus grand avantage d'une amplification du contraste entre les deux bulbes olfactifs. En effet, les bulbes s'inhibent réciproquement, au point d'oblitérer la stimulation du plus faiblement stimulé. Pour une différence d'intensité de 5 à 10%, il s'ensuit une sensation subjective de stimulation unilatérale. Cet effet de contraste n'est pas affecté par les différences qualitatives, entre les courants odorants échantillonnés dans chaque narine; ce contraste est maximum quand les deux stimuli sont délivrés avec un décalage inférieur à la milliseconde. L'olfactomètre de l'invention permet bien d'obtenir cette simultanéité dans l'injection des deux stimuli olfactifs. Les détecteurs thermiques se réchauffent au moment de l'expiration de l'expérimentateur, ils se refroidissent brutalement au moment de son inspiration. Le signal de commande délivré par le détecteur de coïncidence est sensible au refroidissement simultané des deux capteurs thermiques et permet bien de provoquer un escamotage simultané des deux gaines entourant les gicleurs. Les signaux thermiques en provenance des capteurs thermiques sont amplifiés séparément; leur décalage est comparé avec une très faible tolérance de quelques millisecondes dans le détecteur constitué par la porte ET. Il en résulte qu'une véritable mesure de la binarinalité du sujet est ainsi effectuée pour chaque flairage.

Toute évolution vasculaire de la cavité nasale est ainsi prévenue. Il en résulte que les seules mesures qui sont prises en compte, sont celles qui correspondent à des flairages parfaitement bilatéraux et synchrones.

Il est bien évident que dans l'olfactomètre qui vient d'être décrit, les moyens utilisés auraient pu être remplacés par des moyens équivalents, sans sortir du cadre de l'invention.

**Revendications**

1. Olfactomètre différentiel comprenant:
— deux gicleurs (1, 2) situés de part et d'autre d'un support nasal (3) et respectivement associés à un circuit (4) d'alimentation en stimuli olfactifs, chaque gicleur étant constitué par un conduit à orifice calibré
— un dispositif antidiffusion escamotable (5) destiné à occulter les gicleurs pendant des intervalles de temps compris entre les périodes de stimulation nasale;
— un dispositif de détection (7, 8, 9), de la respiration associé à une unité de commande électronique (6) pour commander l'occultation des gicleurs;
— des moyens pour alimenter les gicleurs en permanence caractérisé en ce que le dispositif antidiffusion (5) est constitué, pour chaque gicleur (1, 2), par une gaine (16, 17) entourant le conduit cette gaine présente à sa partie supérieure du côté de l'orifice du conduit, un orifice (18) permettant le passage de ce conduit, tandis que sa partie inférieure évasée par rapport au conduit débouche dans une chambre (19) de pompage permanent, cette gaine étant associée à l'unité de commande électronique (6) par des moyens (20, 21) pour la déplacer linéairement le long du conduit, de manière que le conduit soit entièrement contenu dans la gaine lorsque le détecteur de coïncidence (9) ne délivre aucun signal de commande, et que le conduit émerge de l'orifice de la gaine, lorsque le détecteur de coïncidence délivre un signal de commande d'escamotage.

2. Olfactomètre selon la revendication 1, caractérisé en ce que le dispositif de détection de respiration comprend:
— deux capteurs de température (7, 8) disposés respectivement au voisinage de chaque gicleur (1, 2) et associés à l'unité de commande électronique (6) d'occultation des gicleurs;
— un dispositif détecteur de coïncidence (9) connecté aux deux capteurs (7, 8) et à l'unité de commande électronique (6) qui commande l'escamotage du dispositif antidiffusion (5), lorsque les capteurs présentent un refroidissement simultané.

3. Olfactomètre selon la revendication 1, caractérisé en ce que les moyens pour déplacer les gaines comprennent un moteur de type électromagnétique (20) à déplacements linéaires alternatifs, associé à un élément de couplage (21) des gaines, ce moteur étant commandé par l'unité de commande électronique (6).

4. Olfactomètre selon la revendication 1, caractérisé en ce que les moyens pour déplacer les gaines comprennent, pour chacune de ces gaines, un moteur de type électromagnétique (20, 25) à déplacements linéaires alternatifs, commandé par l'unite de commande électronique (6).

5. Olfactomètre selon la revendication 3, caractérisé en ce que l'unité de commande électronique (6) comprend des moyens (29) pour fixer un délai réglable dans l'application au moteur du signal de commande d'escamotage, et des moyens (30) pour fixer une durée d'escamotage, à partir de la fin de ce délai.

6. Olfactomètre selon la revendication 4, caractérisé en ce que l'unité de commande électronique (6) comprend, pour chacun des moteurs (20, 25), des moyens (29, 32) pour fixer un délai réglable dans l'application au moteur du signal de commande d'escamotage, et des moyens (30, 33) pour fixer une durée d'escamotage, à partir de la fin de ce délai.

7. Olfactomètre selon la revendication 1, caractérisé en ce que le circuit (4) d'alimentation des gicleurs (1, 2) en stimuli olfactifs comprend pour chacun des gicleurs, une cartouche (12, 13) contenant un produit odorant, cette cartouche débouchant d'une part sur le gicleur, et d'autre part, sur des moyens (14) pour alimenter la cartouche par un fluide gazeux à pression réglable.

8. Olfactomètre selon la revendication 2, caractérisé en ce que les capteurs de température (7, 8) sont des thermocouples, ces thermocouples étant respectivement connectés au détecteur de coïncidence (9), par l'intermédiaire d'une amplificateur (10, 11).

9. Olfactomètre selon la revendication 8, caractérise en ce que le détecteur de coïncidence (9) est une porte logique de type ''ET'', dont chaque entrée reçoit un signal de durée réglable.

10. Olfactomètre selon la revendication 9, caractérise en ce qu'un interrupteur (24) est connecté entre la sortie de la porte ''ET'' et l'entrée de l'unité électronique (6) de commande des mesures olfactométriques.

11. Olfactomètre selon la revendication 6, caractérisé en ce que les moyens (29, 32) pour fixer le délai et la durée d'escamotage sont constitués par des bascules monostables à durée de basculement réglable.

12. Olfactomètre différentiel selon l'une quelconque des revendications 10 et 11, caractérisé en ce que le support nasal (3) a une position réglable par rapport aux gicleurs (1, 2).

13. Olfactomètre différentiel selon l'une quelconque des revendications précédentes, caractérisé en ce que les produits odorants ou les mélanges odorants sont véhiculés jusqu'aux gicleurs (1, 2) par deux canalisations séparées.

**Patentansprüche**

1. Differential-Olfaktometer, das aufweist:
—zwei Düsen (1, 2), die beiderseits einer Nasenstütze (3) liegen und jeweils mit einem Versorgungskreis (4) für olfaktorische Stimuli verbunden sind, wobei jede Düse von einer Leitung mit einer kalibrierten Öffnung gebildet wird,
—eine einziehbare Antidiffusionseinrichtung (5), die zum Bedecken der Düsen während Zeitintervallen bestimmt ist, die zwischen den Nasenstimulationsperioden liegen,
—eine Detektoreinrichtung (7, 8, 9) für die Atmung, die mit einer elektronischen Steuereinheit (6) zur Steuerung der Verdeckung der Düsen verbunden ist, und
—Mittel zur ständigen Versorgung der Düsen,
dadurch gekennzeichnet, daß die Antidiffusionseinrichtung (5) für jede Düse (1, 2) von einer Hülse (16, 17) gebildet wird, die die Leitung umgibt, wobei diese Hülse an ihrem oberen Teil an der Seite der Öffnung der Leitung eine Öffnung (18) aufweist, die den Durchgang dieser Leitung gestattet, während ihr unterer Teil, der bezüglich der Leitung ausfallend ist, in eine ständige Pumpenkammer (19) mündet, und wobei diese Hülse mit der elektronischen Steuereinheit (6) durch Mittel (20, 21) zu ihrer linearen Verschiebung längs der Leitung derart verbunden ist, daß die Leitung vollständig in der Hülse aufgenommen ist, wenn der Koinzidenzdetektor (9) kein Steuersignal liefert, und daß die Leitung aus der Öffnung der Hülse austritt, wenn der Koinzidenzdetektor (9) ein Steuersignal zum Einziehen liefert.

2. Olfaktometer nach Anspruch 1, dadurch gekennzeichnet, daß die Ermittlungseinrichtung für die Atmung aufweist:
—zwei Temperaturmeßfühler (7, 8), die jeweils in der Nähe einer jeden Düse (1, 2) angeordnet und mit der elektronischen Steuereinheit (6) für die Verdeckung der Düsen verbunden sind,
—eine Koinzidenzdetektoreinrichtung (9), die mit den beiden Meßfühlern (7, 8) und der elektronischen Steuereinheit (6) verbunden ist, die das Einziehen der Antidiffusionseinrichtung (5) befiehlt, wenn die Meßfühler simultan eine Abkühlung feststellen.

3. Olfaktometer nach Anspruch 1, dadurch gekennzeichnet, daß die Mittel zur Verschiebung der Hülsen einen Motor (20) des elektromagnetischen Typs mit alternativer Linearverschiebung aufweisen, der mit einem Kopplungselement (21) für die Hülsen verbunden ist, und daß dieser Motor durch die elektronische Steuereinheit (6) gesteuert wird.

4. Olfaktometer nach Anspruch 1, dadurch gekennzeichnet, daß die Mittel zur Verschiebung der Hülsen für jede Hülse einen Motor (20, 25) des elektromagnetischen Typs mit alternativer Linearverschiebung aufweisen, der von der elektronischen Steuereinheit (6) gesteuert wird.

5. Olfaktometer nach Anspruch 3, dadurch gekennzeichnet, daß die elektronische Steuereinheit (6) Mittel (29) zur Vorgabe einer regelbaren Zeit beim Anlegen des Steuersignals für das Einziehen an den Motor und Mittel (30) zum Vorgeben einer Einziehzeitdauer ab dem Ende dieser Zeit aufweist.

6. Olfaktometer nach Anspruch 4, dadurch gekennzeichnet, daß die elektronische Steuereinheit (6) für jeden Motor (20, 25) Mittel (29, 32) zur Vorgabe einer regelbaren Zeit beim Anlegen des Steuersignals für das Einziehen an den Motor und Mittel (30, 33) zur Vorgabe einer Einziehzeitdauer ab dem Ende dieser Zeit aufweist.

7. Olfaktometer nach Anspruch 1, dadurch gekennzeichnet, daß der Versorgungskreis (4) der Düsen (1, 2) mit olfaktorischen Stimuli für jede Düse eine Patrone (12, 13) aufweist, die ein Geruchsmittel enthält, und daß diese Patrone einerseits in die Düse und andererseits in Mittel (14) mündet, um die Patrone mit einem gasförmigen Fluid unter einem regelbaren Druck zu versorgen.

8. Olfaktometer nach Anspruch 2, dadurch gekennzeichnet, daß die Temperaturmeßfühler (7, 8) Thermoelemente sind, und daß diese Thermoelemente jeweils unter Zwischenschaltung eines Verstärkers (10, 11) mit dem Koinzidenzdetektor (9) verbunden sind.

9. Olfaktometer nach Anspruch 8, dadurch gekennzeichnet, daß der Koinzidenzdetektor (9) ein logisches UND-Verknüpfungsglied ist, dessen jeweiliger Eingang ein Signal mit regelbarer Zeitdauer erhält.

10. Olfaktometer nach Anspruch 9, dadurch gekennzeichnet, daß ein Unterbrecher (24) zwischen dem Ausgang des UND-Verknüpfungsgliedes und dem Eingang der elektronischen Steuereinheit (6) zur Steuerung der olfaktorischen Messungen vorgesehen ist.

11. Olfaktometer nach Anspruch 6, dadurch gekennzeichnet, daß die Mittel (29, 32) zur Vorgabe der Zeit und der Einziehzeitdauer von monostabilen Kippschaltungen mit regelbarer Kippdauer gebildet werden.

12. Differential-Olfaktometer nach einem der Ansprüche 10 und 11, dadurch gekennzeichnet, daß die Nasenstütze (3) bezüglich den Düsen (1, 2) eine verstellbare Position hat.

13. Differential-Olfaktometer nach einem der vorhergehenden Ansprüche, dadurch gekennzeichnet, daß die Geruchsmittel oder die Geruchsmischungen über zwei gesonderte Leitungen zu den Düsen (1, 2) übertragen werden.

## Claims

1. Differential olfactometer comprising:
—two nozzles (1, 2) located one on each side of a nose support (3) and respectively associated with a supply circuit (4) for olfactory stimuli, each nozzle comprising a conduit with a calibrated orifice,

—a retractable antidiffusion device (5) for covering the nozzles during time intervals between periods of nasal stimulation,

—a respiration detector (7, 8, 9) linked to an electronic command unit (6) for commanding covering of the nozzles, and

—means for permanently feeding the nozzles, characterized in that the antidiffusion device comprises, for each nozzle (1, 2) a sheath (16, 17) surrounding the conduit, said sheath having at its upper part, near the orifice of the conduit, an orifice (18) permitting passage of the conduit, while the lower part, flared in relation to the conduit, opens into a continuously exhaustible chamber (19), said sheath being linked to the electronic command unit (6) by means (20, 21) for linearly displacing it lengthways of the conduit, whereby the conduit is entirely contained within the sheath when the coincidence detector (9) delivers no command signal, and the conduit emerges from the sheath orifice when the coincidence detector delivers a signal commanding retraction.

2. Olfactometer according to Claim 1, characterized in that the respiration detector comprises
—two temperature sensors (7, 8) each located in the vicinity of a respective nozzle and connected to the electronic command unit (6) for covering the nozzles,

—a coincidence detector (9) connected to the two sensors (7, 8) and to the electronic command unit (6) which commands retraction of the antidiffusion device, when the sensors detect a simultaneous cooling.

3. Olfactometer according to Claim 1, characterized in that the means for displacing the sheaths comprise an electromagnetic motor (20) for alternating linear displacement, linked to an element (21) for coupling the sheaths, the motor being controlled by the electronic command unit (6).

4. Olfactometer according to Claim 1, characterized in that the means for displacing the sheaths comprise, for each of the sheaths, a respective electromagnetic motor (20, 25) for alternating linear displacement, controlled by the electronic command unit.

5. Olfactometer according to Claim 3, characterized in that the electronic command unit (6) comprises means (29) for setting a controllable delay in transmission to the motor of the signal commanding retraction, and means (30) for setting a retraction period, at the end of said delay.

6. Olfactometer according to Claim 4, characterized in that the electronic command unit (6) comprises, for each of the motors (20, 25) means (29, 32) for setting a controllable delay in the transmission to the motor of the signal commanding retraction, and means (30, 33) for setting a retraction period, at the end of said delay.

7. Olfactometer according to Claim 1 characterized in that the circuit (4) supplying the nozzles (1, 2) with olfactory stimuli comprises, for each of the nozzles, a cartridge (12, 13) containing an odoriferous product, said cartridge leading on the one hand to the nozzle, and on the other hand to a means (14) for supplying the cartridge with a gas under controllable pressure.

8. Olfactometer according to Claim 2 characterized in that the temperature sensors (7, 8) are thermocouples, coupled to the coincidence detector (9) by respective amplifiers (10, 11).

9. Olfactometer according to Claim 8, characterized in that the coincidence detector (9) is an AND-type logic gate, each of whose inputs receives a signal of controllable duration.

10. Olfactometer according to Claim 9, characterized in that an interruptor (24) is located between the output of the AND gate and the input of the electronic command unit (6).

11. Olfactometer according to Claim 6 characterized in that the means (29, 32) for setting the delay and the duration of retraction are monostable flip-flops having a controllable operating time.

12. Differential olfactometer according to either of Claims 10 to 11, characterized in that the nose support (3) has an adjustable position with respect to the nozzles (1, 2).

13. Differential olfactometer according to any one of the preceding Claims characterized in that the odoriferous products or odoriferous mixtures are conducted to the nozzles (1, 2) by two separate channels.

FIG. 1

# FIG. 2

FIG. 3

FIG. 4

FIG. 5

FIG. 6

4